# EUROPEAN PATENT APPLICATION

(11) **EP 3 828 573 A1**
(43) Date of publication of application: **02.06.2021**
(21) Application number: 19211918.8
(22) Date of filing: 27.11.2019
(51) Int. Cl.: G01R 33/38, G01R 33/385, A61B 5/055

(54) **V-SHAPED GRADIENT SYSTEM FOR A MAGNETIC RESONANCE IMAGING SYSTEM**

(71) Applicant: Siemens Healthcare GmbH, 91052 Erlangen (DE)
(72) Inventor: Popescu, Stefan, 91056 Erlangen (DE); Vester, Markus, 90471 Nürnberg (DE)

(57) **Abstract**

The invention describes a gradient system for a magnetic resonance imaging system comprising a number of pairs of gradient coils (25x, 25y, 25z), wherein the gradient coils (25x, 25y, 25z) of each pair comprise a central plane (23) and are arranged at opposite sides of an examination area (M1, M2, M3, M4, M5, M6) such that the central planes (23) of the two gradient coils (25x, 25y, 25z) of a pair are at an angle greater than 10° to another so that the gradient system (20) is V-shaped.

The invention further describes a magnetic resonance imaging system with such gradient system.

## Description

The invention describes a Gradient system for a magnetic resonance imaging system ("MRI-system"), especially for a MRI-system with two or more examination areas, and such MRI-System.

For more than four decades, the principle of magnetic resonance imaging ("MRI") has been used for imaging and other measurements. Despite this long time and the importance of this method of measurement, only two magnet designs are currently used for clinically used MRI systems or MRI scanners: C magnet forms and solenoids. Operation of this type of MRI scanner still are problematic for the clinical workflow.

The most serious problems occur with regard to the extensive stray magnetic fields around these scanners. In order to cope with this problem and avoid accidents and damage, the hospital administration must delineate a strictly controlled area within and in the vicinity of the MRI examination rooms by limiting the access of people and equipment. Damage can occur if metallic or magnetic parts are attracted by the strong magnets of the MRI scanner and accelerated in the direction of the scanner volume.

Another problem is that the MRI scanners, which use a solenoid-magnet design, "enclose" patients in a narrow patient tunnel, which in particular can cause claustrophobia. This claustrophobia may be so strong in some patients that no MRI scan can be performed. Moreover, due to the narrowness of the examination tunnel, the access of the medical staff to the patient is severely restricted, which is unfavorable for interventional or therapeutic procedures, in particular with regard to real-time MRI imaging.

Typically, MRI scanners use a self-shielded, solenoid-type superconducting magnet to reduce the strength of the leakage magnetic field resulting from the coil of the basic field magnet. An actively shielded basic field magnet is much more expensive than an unshielded one. In addition, the shield coils reduce the efficiency of the basic magnetic field that can be used for measurements in an examination tunnel. Active shielded magnets have a larger diameter (about 220 cm) than unshielded magnets (about 145 cm).

Alternative designs for MR scanners use a C-shaped magnet. This can be either a permanent magnet or an electromagnet consisting of two Helmholtz coils. The C-shaped magnets have two magnetic pole pieces which create a vertical basic magnetic field in their space. An analogous structure is a portal magnet, which is mechanically more robust, and in some embodiments can also be realized with superconducting Helmholtz coils. The C-shape and the portal magnets have the advantage of open access to the patient and additionally reduce claustrophobic feelings. However, such a structure requires a very robust mechanical construction to counteract the enormous magnetic attraction force between the two opposed basic field magnets. To reduce the spread of stray magnetic fields, these magnet architectures typically use an iron yoke to guide and close the magnetic field lines outside the imaging volume. The iron yoke is one of the most cost-effective shields. The disadvantage of such a yoke is the big size, weight and volume of the MR scanner.

One approach to solve these problems has been introduced a short time ago. This approach is based on an MRI system with a toroidal magnetic field. Unlike the prior art of MR magnets that use solenoid or Helmholtz-pair magnet coils, the toroid coils tend to confine the magnetic field inside the torus with only a small and not so far reaching stray magnetic field. This system not only overcomes the problems of stray magnetic field and a light-weight construction, it also offers the opportunity to realize two or more examination areas in one single MRI-system. An example for such MRI-system is a basic field magnet arrangement with three, four, six or eight (e.g. identical) basic field magnet segments arranged in a star shape about a central axis with a rotational symmetry (e.g. 60° for six magnets and six examination areas). The basic magnetic field has a main direction which runs in the form of a toroidal magnetic field.

There are local gradient systems with coil pairs arranged parallel left and right of a patient. However, although such known gradient systems can also be used for these new MRI-systems, there is currently no gradient system working in an optimal way together with these MRI-systems.

It is the object of the present invention to improve the known MRI-systems to facilitate an improved gradient system, especially for producing spatially non-constant magnetic fields, preferably for MRI-systems with more than one examination areas.

This object is achieved by a gradient system according to claim 1, and a magnetic resonance imaging system according to claim 8.

A gradient system according to the invention for a magnetic resonance imaging system comprises a number of pairs of gradient coils, wherein the gradient coils of each pair comprise a central plane and are arranged at opposite sides of an examination area such that the central planes of the two gradient coils of a pair are at an angle greater than 10° to another so that the gradient system is V-shaped.

Although the gradient system is very advantageous for star-shaped magnet arrangements, it is also advantageous for conventional MRT-systems.

The expression "pairs of gradient coils" means that there are two gradient coils for each gradient axis. Typically, the gradient system creates gradients in X, Y and Z direction to realize a gradient vector with X, Y and Z contributions. Thus, it is preferred that there is a pair of X gradient coils, a pair of Y gradient coils and a pair of Z gradient coils. It is clear that two gradient coils designed to create the same gradient (and being arranged on different sides of an examination area) are forming a pair.

To clarify the position in a 3D coordinate system, there is a reference to a central plane of the gradient coils. The central plane of a coil is the plane of the loops of the coil (or at least an averaged middle plane). Looking at a planar gradient coil, the central plane is the plane of the coil, looking at a Helmholtz coil, the central plane is the plane of the windings of the coil, wherein the magnetic field is perpendicular to the plane.

The gradient coils of each pair (i.e. the X, Y, and/or Z gradient coils) are arranged at opposite sides of an examination area. The examination area of a gradient system is the area between the gradient coils, since at normal use in an MRT scanner, the examination area for the MRI examination would be between the gradient coils.

The gradient coils are arranged in a special way: They are not aligned parallel as in the state of the art, but such that the central planes of the two coils of a pair are at an angle greater than 10° to another. Thus, the gradient system is V-shaped. For better understanding it is defined here that the "front side" of the gradient system is the mouth of the V-shape and the "back side" is the opposite side, where the gradient coils of a pair are nearest to each other. The Vector of the X-Axis points to the front side of the gradient system.

Surely, the gradient system should also comprise further components that the gradient systems of the state of the art also comprise for an optimal functioning. These are e.g. dedicated gradient power amplifiers for each axis GPAx, GPAy and GPAz, shim coils or holding structures.

Such gradient system has the advantage that it produces a special gradient field that can be used for spatial resolvable gradients. However, it is very advantageous for MRIscanners with inclined arrangements of basic field magnets as e.g. star-shaped arrangements, as said above.

A magnetic resonance imaging system comprises at least one examination area, preferably two or more examination areas, and a gradient system according to the invention in at least one examination area. A preferred MRI-scanner of such magnetic resonance imaging system comprises a inclined arrangement of basic field magnets, e.g. a star-shaped arrangement. MRI scanners with a toroidal MR scanner architecture are particularly preferred. In a star shaped arrangement of basic field magnets with a toroidal magnetic field, the front side of the gradient system should point to the outside of the toroid magnetic field.

Particularly advantageous embodiments and features of the invention are given by the dependent claims, as revealed in the following description. Features of different claim categories may be combined as appropriate to give further embodiments not described herein.

According to a preferred gradient system, the gradient coils of each pair are arranged in a mirrored arrangement at the opposite sides of the examination area such that the central planes of the two gradient coils of a pair are symmetrical to an angle bisector. An angle bisector divides the angle spanned by the respective central planes into two angles with equal measures. A preferred gradient system has the shape of a isosceles triangle (with an open hypothenuse).

According to a preferred gradient system, the gradient coils are bi-planar gradient coils. This hat the advantage that the gradient system does not need much space.

It is preferred that the central planes of gradient coils on one side of the examination area are parallel to each other, especially in the case the gradient coils are bi-planar gradient coils.

It is preferred that the gradient system comprises two or three pairs of gradient coils (e.g. X, Y and Z gradient coils), wherein all pairs of gradient coils are arranged in the same angle to another, i.e. the angle between the central planes are equal.

According to a preferred gradient system, a number of gradient coils is formed as local coils, preferably as local gradient coils for head imaging. Preferably the gradient coils are integrated into and/or are mechanically attached to the headrest of a patient chair.

Alternatively or additionally, it is preferred that a number of gradient coils is formed to cover a side of a whole field-of-view (or at least a part of the FoV) of an examination area, preferably being mechanically and/or permanently attachable to an MRI-scanner. Preferably, the gradient coil system has two building blocks symmetrically located at the right side (say block 1) and the left side (say block 2) of the examination area. The right block 1 and the left block 2 are preferably mirrored paired. Each block preferably integrates at least one gradient coil for one axis. Generally, each block integrates three gradient coils for all three axes. This means that the block 1 comprises a stack of planar gradient coils, e.g. the right half of the X, Y and Z gradient coils. Especially with a V-shaped basic-magnet arrangement, an (or each) examination area has a V-shaped gradient coil system attached to the basic field magnets and comprising the two blocks. Such V-shaped architecture of gradient coils makes better use of the magnetic field space available within the examination regions that is shaped like a triangular or trapezoidal prism. As for the local gradients system, each block (1, 2) consists of a stack of planar gradient coils, e.g. each block integrates one half of the X, Y and Z gradient coil pairs.

A preferred gradient system comprises magnetic field shim coils and/or active shielding coils, wherein these coils are preferably arranged parallel to the gradient coils (i.e. the central plane of these coils is parallel to the central plane of the gradient coils). Regarding the preceding example, the gradient system blocks (1,2) could further integrate magnetic field shim coils and/or active shielding coils so designed to attenuate the stray gradient fields outside the imaging volume that would otherwise penetrate the adjacent examination regions and/or imaging volumes.

According to a preferred gradient system, a gradient coil is formed by a plurality of loops of a conductor. It is clear that preferably only one long conductor is wound into a number of loops, however, there could also appear open loops that are connected to another. In the following, the loops of a coil are designated as a "set of loops", wherein any references to moving actions are meant to be understood as changes of following loops. The following designs are preferred (alternatively or additionally):
The loops of a gradient coil for the X-gradient comprising two sets of contrarotating loops adjacent in X-direction, preferably wherein the radius of a set of loops increases, with the outer conductors in X-direction (the front and the back side) essentially remaining at the sides of the gradient coil. This means that the shape of such coil reminds one of a butterfly

The loops of a gradient coil for the Y-gradient comprising two sets of contrarotating loops adjacent in Y-direction, preferably wherein the radius of a set of loops increases, with the outer conductors in Y-direction essentially remaining at the sides of the gradient coil (the sides perpendicular to the front and the back side). This means that the coil may look as the coil for the X gradient only rotated 90°.

The loops of a gradient coil for the Z-gradient comprising a set of increasing loops, preferably wherein the center of the loops essentially remain in the center of the gradient coil. This means that the coils may be coaxial but growing bigger, at least in X-direction.

In the following, more preferred special designs of coils are described. This special design results in a field that increases in the direction of the mouth of the V-shape (to the front side) of the gradient system.

Regarding the gradient coil for the X-gradient, the distance of the field-relevant conductors of a set of loops steadily decreases at least in the direction of the aperture of the V-shape (in direction to the front side) of the gradient system. The field relevant conductors are these part of the loops that determine the magnetic field of a gradient.

Regarding the gradient coil for the Z-gradient, the distance of the field-relevant conductors of a set of loops steadily decreases at least in the direction of the aperture of the V-shape (in direction to the front side) of the gradient system.

Regarding the gradient coil for the Y-gradient, the radius of a set of loops increases in X-direction as well as in Y-direction with the outer conductors at the aperture of the V-shape essentially remaining at the side of the aperture (i.e. at the front side) as well as at the sides perpendicular to the front side.

Various hardware or software tools can be used to further fine-tune these wire patterns in order to meet some additional constrains such as the gradient linearity, to reduce the stray fields, the amplitude of mechanical vibrations and the level of acoustic noise or peripheral nerve stimulation.

The described wire pattern distributions could eliminate the inherent non-linearity of the gradient fields along a radial direction or along the local X-axis. This non-linearity only results with V-shaped gradient coils when the wire pattern density along the X-axis is approximately constant.

Special loop-shaping as described may eliminate non-linear components along the X-axis for the X and the Z gradient coils by modifying the wire spacing along the X-axis from a constant one to a more quadratic one, with the wire density increasing approximatively quadratically with the radial distance to the axis of symmetry (e.g. of a toroid basic magnet). For the Y gradient coils an exemplary solution adds an additional wire distribution having constant spacing along the X-axis. This is similar to the wire patterns for the magnet coil used for generating the static magnetic field Bo.

According to a preferred magnetic resonance imaging system, the gradient system comprises a number of gradient coils covering a side (or at least a part of a side) of a whole field-of-view of an examination area, preferably being mechanically and/or permanently attached to the an MRI-scanner of the magnetic resonance imaging system.

According to a preferred magnetic resonance imaging system, central planes of gradient coils of the gradient system on at least one side of the examination area are arranged parallel to basic field magnets of a MRI-scanner of the magnetic resonance imaging system. Thus, the MRI-scanner has inclined basic field magnets and the gradient system follows the arrangement of the basic field magnets.

A preferred opening angle of the V-shape of the gradient system is 120° (e.g. for a star shaped MRI scanner with 3 examination areas), 90° (e.g. for a star shaped MRI scanner with 4 examination areas), 72° (e.g. for a star shaped MRI scanner with 5 examination areas), 60° (e.g. for a star shaped MRI scanner with 6 examination areas) or 45° (e.g. for a star shaped MRI scanner with 8 examination areas).

According to a preferred magnetic resonance imaging system, the magnetic resonance imaging system is designed such that the gradient systems in an examination area, preferably in each examination area, operates asynchronously and/or independent of the gradient system in another examination area of the magnetic resonance imaging system.

Preferably, the gradient system comprises a central control unit that is designed to coordinate all gradient activities, preferably the independent operation of different gradient systems, especially even the minimization and/or the correction of cross-interference terms between the gradient systems. The term "working independently" means that the MR sequences running in the examination regions are not necessarily identical or synchronized or interleaved.

Other objects and features of the present invention will become apparent from the following detailed descriptions considered in conjunction with the accompanying drawings. It is to be understood, however, that the drawings are designed solely for the purposes of illustration and not as a definition of the limits of the invention.
FIG 1 shows a simplified MRI system according to an embodiment of the invention.
FIG 2 shows an exemplary embodiment of a magnetic resonance tomography system with a star-shaped basic field magnet arrangement.
FIG 3 shows a detailed schematic representation of the individual basic field magnet segments of a star-shaped basic field magnet arrangement.
FIG 4 shows a further exemplary embodiment of a magnetic resonance scanner with two examination areas.
FIG 5 shows a schematic example of a gradient system according to the invention.
FIG 6 shows a schematic representation of a planar coil for the X-gradient according to the invention.
FIG 7 shows a schematic representation of a planar coil for the Y-gradient according to the invention.
FIG 8 shows a schematic representation of a planar coil for the Z-gradient according to the invention.
FIG 9 shows an alternative winding for a gradient coil for the X-gradient.
FIG 10 shows an alternative winding for a gradient coil for the Y-gradient.
FIG 11 shows an alternative winding for a gradient coil for the Z-gradient.

In the diagrams, like numbers refer to like objects throughout. Objects in the diagrams are not necessarily drawn to scale.

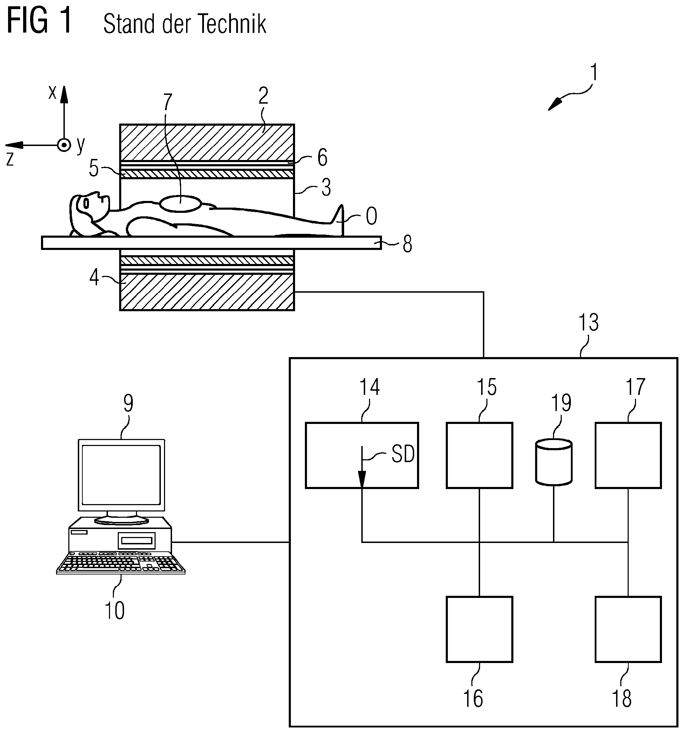

FIG 1 shows a schematic representation of a magnetic resonance imaging system 1 ("MRI-system"). The MRI system 1 includes the actual magnetic resonance scanner (data acquisition unit) 2 with an examination space 3 or patient tunnel in which a patient or test person is positioned on a driven bed 8, in whose body the actual examination object O is located.

The magnetic resonance scanner 2 is typically equipped with a basic field magnet system 4, a gradient system 6 as well as an RF transmission antenna system 5 and an RF reception antenna system 7. In the shown exemplary embodiment, the RF transmission antenna system 5 is a whole-body coil permanently installed in the magnetic resonance scanner 2, in contrast to which the RF reception antenna system 7 is formed as local coils (symbolized here by only a single local coil) to be arranged on the patient or test subject. In principle, however, the whole-body coil can also be used as an RF reception antenna system, and the local coils can respectively be switched into different operating modes.

The basic field magnet system is designed in a typical manner so that it generates a basic magnetic field in the longitudinal direction of the patient, i.e. along the longitudinal axis of the magnetic resonance scanner 2 that proceeds in the z-direction. The gradient system 6 typically includes individually controllable gradient coils in order to be able to switch (activate) gradients in the x-direction, y-direction or z-direction independently of one another.

The MRI system 1 shown here is a whole-body system with a patient tunnel into which a patient can be completely introduced. However, in principle the invention can also be used at other MRI systems, for example with a laterally open, C-shaped housing, as well as in smaller magnetic resonance scanners in which only one body part can be positioned.

Furthermore, the MRI system 1 has a central control device 13 that is used to control the MRI system 1. This central control device 13 includes a sequence control unit 14 for measurement sequence control. With this sequence control unit 14, the series of radio-frequency pulses (RF pulses) and gradient pulses can be controlled depending on a selected pulse sequence.

To output the individual RF pulses of a pulse sequence, the central control device 13 has a radio-frequency transmission device 15 that generates and amplifies the RF pulses and feeds them into the RF transmission antenna system 5 via a suitable interface (not shown in detail). To control the gradient coils of the gradient system 6, the control device 13 has a gradient system interface 16. The sequence control unit 14 communicates in a suitable manner with the radio-frequency transmission device 15 and the gradient system interface 16 to emit the pulse sequence.

Moreover, the control device 13 has a radio-frequency reception device 17 (likewise communicating with the sequence control unit 14 in a suitable manner) in order to acquire magnetic resonance signals (i.e. raw data) for the individual measurements, which magnetic resonance signals are received in a coordinated manner from the RF reception antenna system 7 within the scope of the pulse sequence.

A reconstruction unit 18 receives the acquired raw data and reconstructs magnetic resonance image data therefrom for the measurements. This reconstruction is typically performed on the basis of parameters that may be specified in the respective measurement or control protocol. For example, the image data can then be stored in a memory 19.

Operation of the central control device 13 can take place via a terminal 10 with an input unit and a display unit 9, via which the entire MRI system 1 can thus also be operated by an operator. MR images can also be displayed at the display unit 9, and measurements can be planned and started by means of the input unit (possibly in combination with the display unit 9), and in particular suitable control protocols can be selected (and possibly modified) with suitable series of pulse sequence PS as explained above.

The MRI system 1 according to the invention, and in particular the control device 13, can have a number of additional components that are not shown in detail but are typically present at such systems, for example a network interface in order to connect the entire system with a network and be able to exchange raw data and/or image data or, respectively, parameter maps, but also additional data (for example patient-relevant data or control protocols).

The manner by which suitable raw data are acquired by radiation of RF pulses and the generation of gradient fields, and MR images are reconstructed from the raw data, is known to those skilled in the art and thus need not be explained in detail herein.

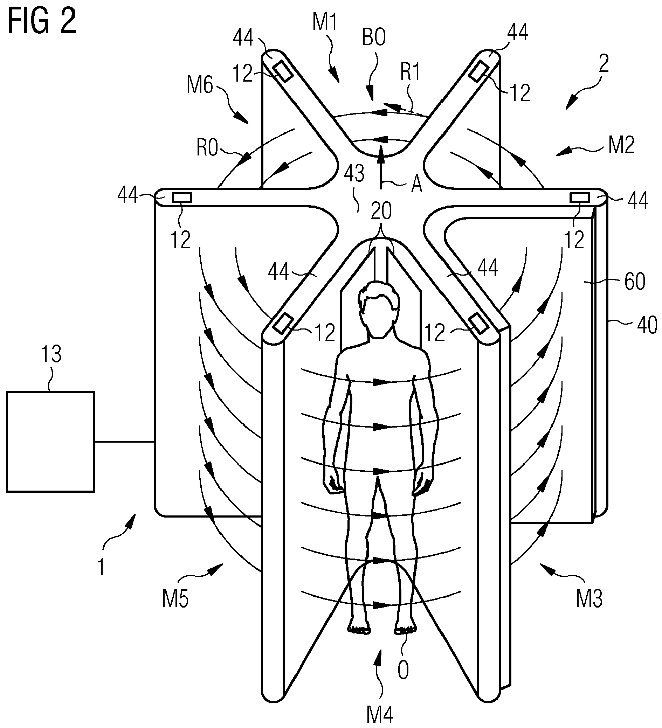

FIG. 2 shows an exemplary embodiment of a magnetic resonance tomography system 1 with a star-shaped basic field magnet arrangement 40.

Shown here is a magnetic resonance scanner 2, the function of which can be controlled by a control device 13. The control device 13 can in principle be constructed in a similar manner and have the same components as the control device 13 in a conventional MR system according to FIG. 1. Likewise, it can also have a suitable terminal or the like (which is not shown here) .

The basic field magnet arrangement 40 of the magnetic resonance scanner 2 in this figure comprises six (here identical) basic field magnet segments 44, which in this embodiment are arranged in a star shape about a central axis A with a rotational symmetry of 60°. The basic magnetic field B0 indicated by arrows has a basic field main direction R0, which runs in the form of a circle or a toroidal magnetic field.

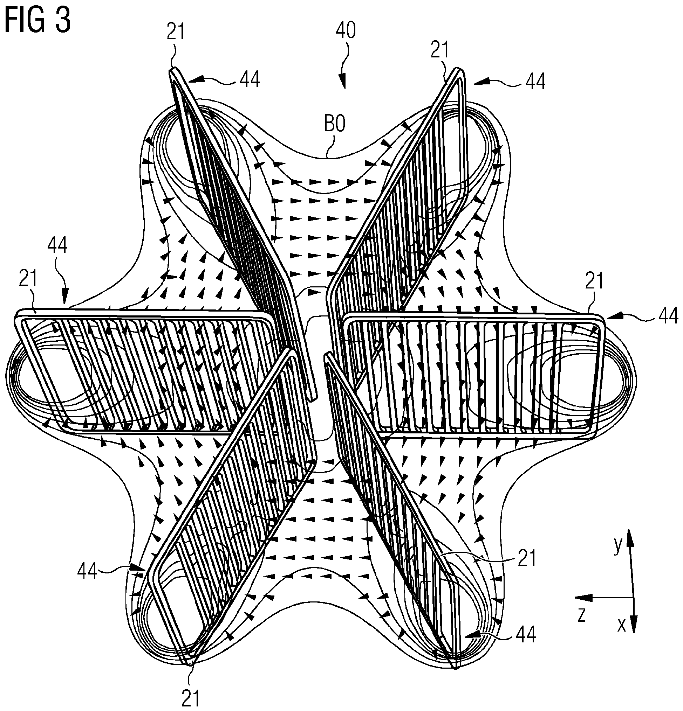

FIG. 3 shows a detailed schematic representation of the individual basic field magnet segments 44 of a star-shaped basic field magnet arrangement 40. Six coil-arrangements can be seen here as basic field magnet segments 44 of the basic field magnet arrangement 40.

It should be noted that in FIG 3 the basic magnetic field B0 doesn't form circles, but hexagonal contours in each examination area M1, M2, M3, M4, M5, M6. The field lines of the basic magnetic field B0 form parallel lines.

This "six-pack" toroidal MRI-scanner allows scanning up to six patients simultaneously in six imaging volumes (examination areas) wherein the homogeneity of the magnet field Bo is high enough for conducting MR imaging sequences as known from prior art. This scanner is supposed to acquire raw data and to reconstruct MR images by acquisition and image reconstruction methods as explained in the course of FIG 1.

For each imaging volume there is a local coordinate system XYZ associated therewith as exemplary depicted here only for one imaging volume. The local Z-axis is running parallel to and pointing in the same direction with the static magnetic field B0. The Y-axis is parallel to the rotational symmetry axis of the six-pack magnet system, while the X-axis corresponds to the radial direction pointing from the center of symmetry outwards from the magnet.

Arrows and iso-lines depict the overall distribution of the static magnetic field B0 within a cross sectional planar cut through the middle of the magnet. The local magnetic field vectors depicted by small arrows show the distribution of the local magnetic field magnitude (arrow size) and its direction (arrow orientation).

A significant advantage of such a symmetrical arrangement is the structural stability when the basic magnetic field B0 is switched on. The magnetic forces between the individual basic field magnetic segments 44 cancel each other out in the direction of the main magnetic field direction R0. Each basic field magnet segment 44 is attracted by its two neighbors, each with the same force. The resulting forces act inwardly towards the pillar 43 and can be compensated there very well by appropriate structural reinforcements.

Such a magnetic resonance imaging system 1 with a basic field magnet arrangement 40 according to figures 2 and 3 permits measurements at six different examination areas M1, M2, M3, M4, M5, M6 (see FIG 2), wherein in the illustrated example a measurement of an object O (a patient as shown or an inanimate object) occurs at examination area M4, wherein a patient stands here upright on vertical walls of the basic field magnet arrangement 40. Theoretically, measurements could take place simultaneously at all six examination areas M1, M2, M3, M4, M5, M6. A central pillar 43 holds the basic field magnet segments 44 in place and may also comprise technical components, such as e.g. the electrical connections or even the power supply.

In each case, measuring devices 12 (only shown symbolically) or the components respectively required for this purpose at examination area M1, M2, M3, M4, M5, M6, such as an HF coil, can be located at examination areas M1, M2, M3, M4, M5, M6 Transmitting coil of an RF transmission system, an RF reception coil of an RF reception system and/or a common RF transmission / reception coil. Likewise, this may include gradient and/or shim coils. All of these components can be controlled coordinated by the common control device 13.

Of course, a magnetic resonance scanner 2 may also have more than six examination areas M1, M2, M3, M4, M5, M6, its height may be lower, or it may be designed for examining small areas of the body, e.g. for head examinations or examinations of the extremities, the female breast, the prostate, the liver, kidneys or other organs. The star-shaped basic field magnet arrangement 40 could also be positioned lying.

An example for a gradient system 20 according to the invention is shown in one examination area M4 around the head of the patient. The V-shape of the gradient system 20 follows the angle between two basic field magnet segments 44, i.e. 60°.

Another example for a gradient system 20 according to the invention is shown in an adjacent examination area M3. This gradient system 20 comprises large gradient coils that covers the whole field-of-view of the examination area M3 and is mechanically and permanently attached to the basic field magnet segments 44 of the MRI scanner 2. Each examination area M1, M2, m3, M4, M5, M6 may have such a V-shaped gradient system 60 attached thereto and consisting in two blocks: the right-block and the mirrored paired left-block. The V-shaped architecture of gradient systems 60 according to the invention makes better use of the magnetic field space available within the examination regions that is shaped like a triangular or trapezoidal prism. As for the local gradient system 20, each block consists of a stack of planar gradient coils 25x, 25y, 25z (see following figures), e.g. each block integrates one half of the X, Y and Z gradient coil pairs. Furthermore, the blocks could also integrate magnetic field shim coils and/or active shielding coils so designed to attenuate the stray gradient fields outside the imaging volume that would otherwise penetrate the adjacent examination regions and/or imaging volumes. The gradient system 20 may further include dedicated gradient power amplifiers for each axis GPAx, GPAy and GPAz and for each examination region, preferably all being controlled by a common central unit.

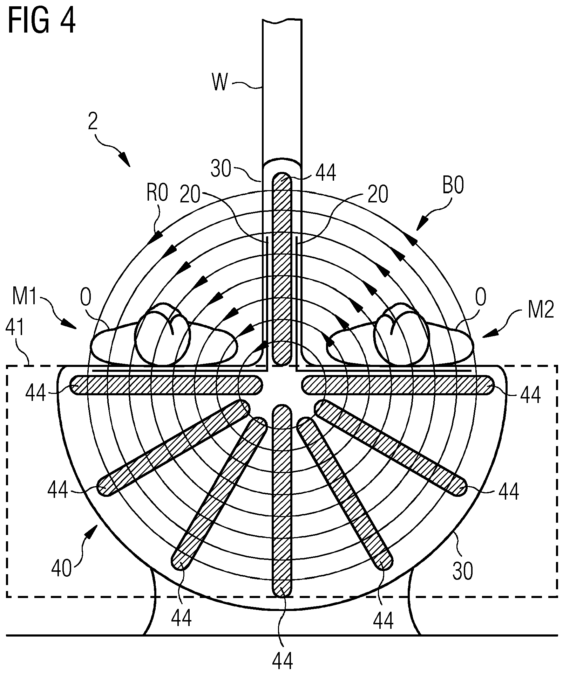

FIG. 4 shows a further exemplary embodiment of a magnetic resonance scanner 2 with two examination areas M1, M2. Here, only the lower half of the basic field magnet arrangement 40 is designed star-shaped as a group 41 of basic field magnet segments 44 and another basic field magnet segment 44 projects upwards and serves both for guiding the basic magnetic field B0 as well as part of a wall W between two examination area M1, M2, on which there are two patients as objects O to be examined. In the illustration it can be seen that the lower part of the wall W between the two patients is formed by the housing wall 30 of the magnetic resonance scanner 2, into which the basic field magnetic segment 44 is integrated between the examination area M1, M2. The wall W can serve not only as a privacy screen but also as an acoustic shield or RF shield.

The basic magnetic field B0 of this magnetic resonance scanner 2 becomes weaker toward the outside, which can be used for location coding, and is homogeneous in the longitudinal direction (orthogonal to the image plane). It is basically the same in shape in the two examination area M1, M2, with the only difference being that the course (in one direction through the surface on which the patient O is lying) is reversed. Again, the dimensions of the magnetic resonance scanner 2 can be chosen quite different.

The basic magnetic main field direction R0 is also circular here. A special feature of this embodiment is that the patients O are not in a narrow space, but can look freely to the ceiling. The inhomogeneity in the basic magnetic field B0, which is usually caused by the curvature, can be used, as mentioned, for spatial encoding resolution in one spatial direction.

Due to its open design and the toroidal magnetic field, this arrangement allows easy and little restricted access to the patient. As a result of the symmetric construction, magnetic forces are largely compensated as in FIG 2 or diverted into areas which can be reinforced structurally well.

An example for a gradient system 20 according to the invention is shown in both examination areas M1, M2. The V-shape of the gradient system 20 again follows the angle between two basic field magnets 44, i.e. here 90°.

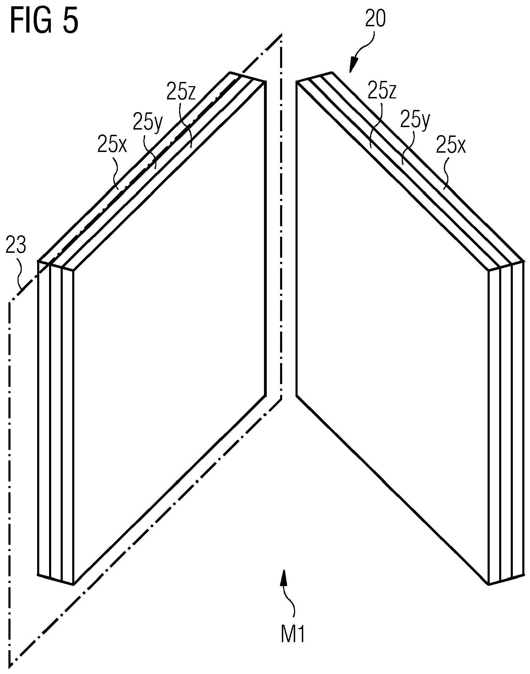

FIG 5 shows a schematic example of a gradient system 20 according to the invention. This gradient system 20 comprises a number of pairs of gradient coils 25x, 25y, 25z, wherein the gradient coils 25x, 25y, 25z of each pair comprise a central plane 23 and are arranged at opposite sides of an examination area M1 such that the central planes 23 of the two gradient coils 25x, 25y, 25z of a pair are at an angle of here about 60° to another so that the gradient system 20 is V-shaped.

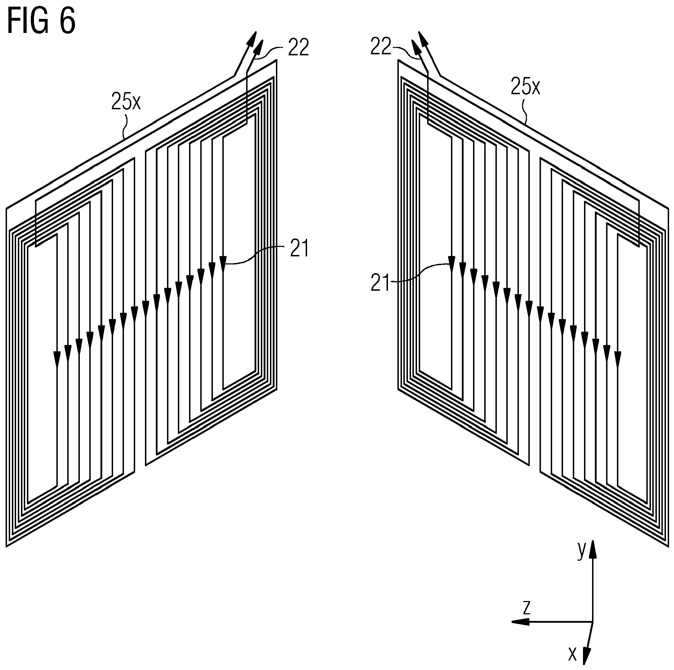

Figures 6, 7 and 8 show schematic representations of planar gradient coils 25x, 25y, 25z for the X-gradient, the Y-gradient and for the Z-gradient. The gradient coils 25x, 25y, 25z are made from current conductors 21. that may be connected to a gradient power supply (not shown) with power connectors 22. The gradient system 60 should comprise all three gradient coils 25x, 25y, 25z for producing a gradient vector with contributions in X-, Y- and Z-direction (see. e.g. FIG 5). The arrows at the conductors 21 always show the predefined direction of the electric current.

FIG 6 shows the gradient coils 25x for the X-gradient. They are shaped such that the radius of following loops increases perpendicular to the field relevant conductors 21 (here the central conductors 21 with the arrow pointing to the bottom). The center of following loops moves in the direction of the center of a gradient coil 25x.

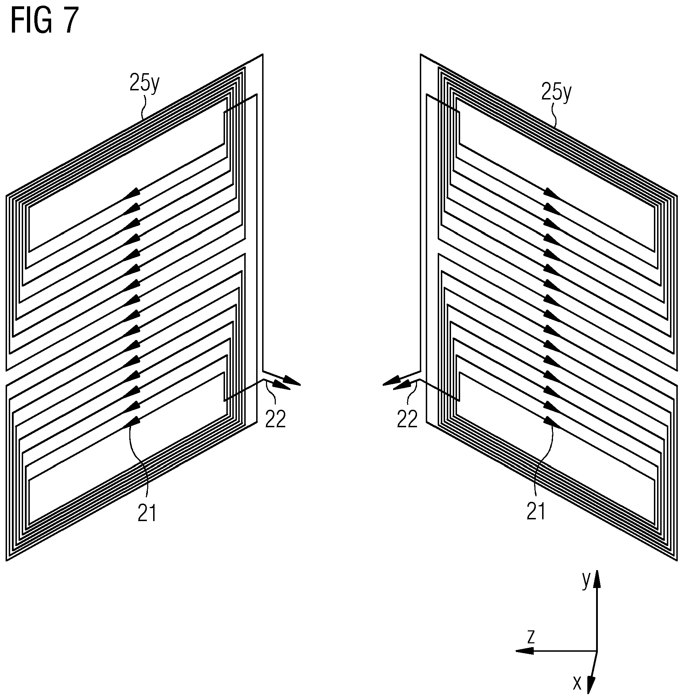

FIG 7 shows the gradient coils 25y for the Y-gradient. They are shaped such that the radius of following loops increases perpendicular to the field relevant conductors 21 (here the central conductors 21 with the arrow pointing to the left/right). The center of following loops moves in the direction of the center of a gradient coil 25y.

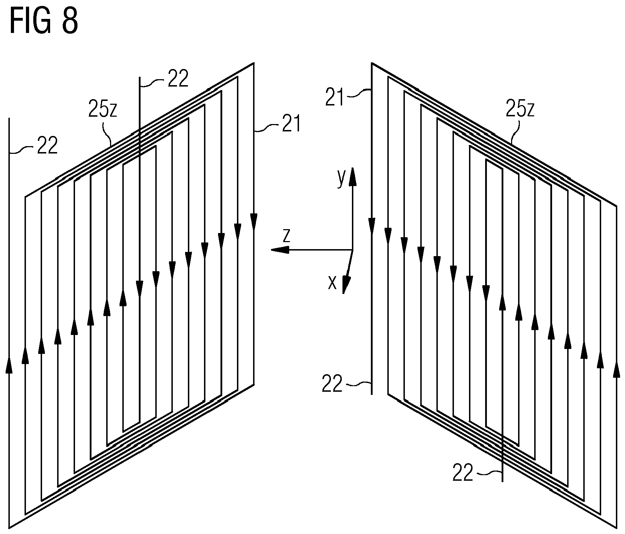

FIG 8 shows the gradient coils 25z for the Z-gradient. They are shaped such that the radius of following loops increases perpendicular to the field relevant conductors 21 (here the conductors 21 with the arrow pointing to the bottom). The center of following loops stays essentially in the center of a gradient coil 25z.

With the wire patterns as depicted by figures 6, 7 and 8, the generated gradient fields would be slightly non-linear along a radial direction or along the local X-axis due to the V-shape of the gradient system 20. This is not a serious limitation for this solution as the prior art of MRI provides various image reconstruction methods that accommodate for non-linear gradient fields. Additionally, MR imaging using non-linear gradients may provide further advantages as variable spatial resolution, faster data acquisition with non-linear signal encoding and feebler peripheral nerves stimulation.

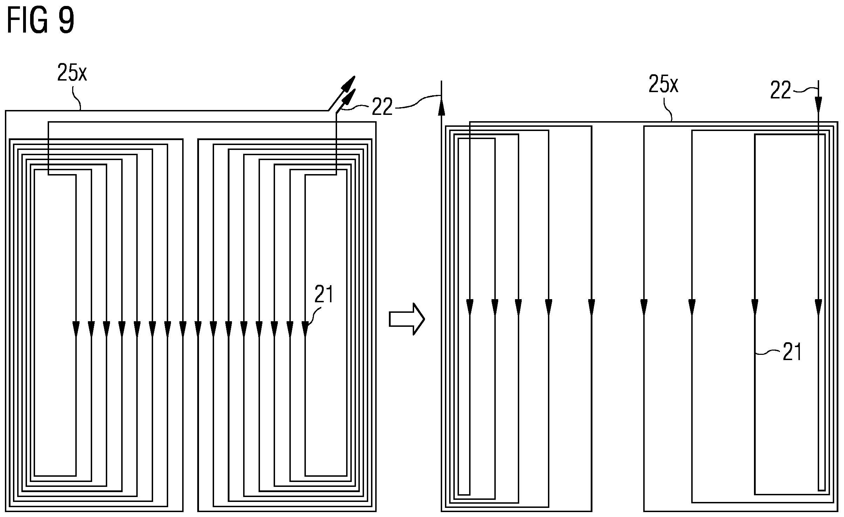

Figures 9, 10 and 11 show alternative windings for the gradient coils 25x, 25y, 25z. On the left there is shown a gradient coil 25x, 25y, 25z as already shown in figures 6, 7 and 8 as reference and on the right a gradient coil 25x, 25y, 25z with an alternative winding. Since the coils are arranged in a V-shape, the magnetic field of the left coils, producing a linear gradient field, gets weaker in the direction to the x-axis (the mouth of the V, i.e. the front side). The right coils 25x, 25y, 25z produce a linear gradient field in V-shaped configuration due to the increasing distance between conductors 21 from the left to the right. For achieving a constant gradient field, the left side of the right gradient coil 25x, 25y, 25z should point in direction of the X-axis.

FIG 9 shows an alternative winding for a gradient coil 25x for the X-gradient. It is shaped such that the radius of following loops increases perpendicular to the field relevant conductors 21 (here the central conductors 21 with the arrow pointing to the bottom) with a non-constant distance between adjacent conductors. The center of following loops moves in the direction of the center of the gradient coil 25x. Thus, the distance of the field-relevant conductors 21 of a set of loops steadily decreases at least in the direction to the front side of the gradient system 2.

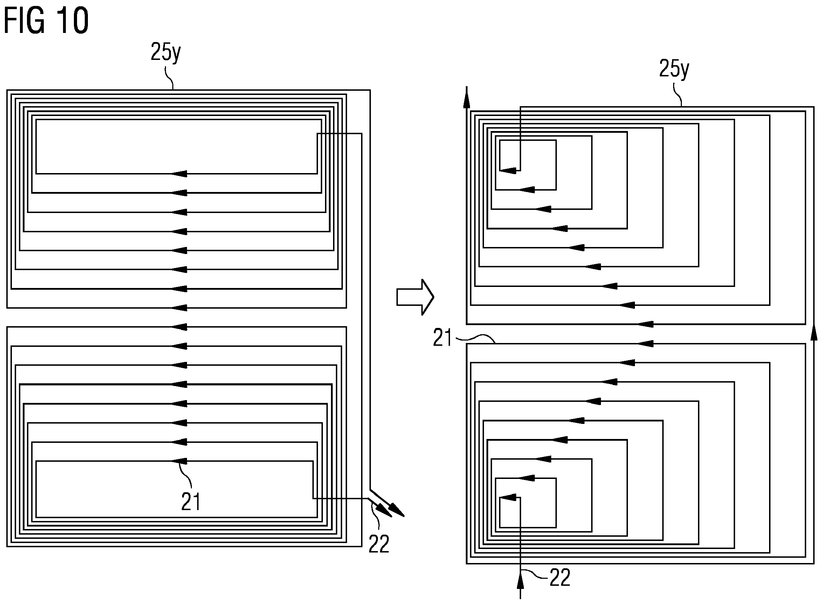

FIG 10 shows an alternative winding for a gradient coil 25y for the Y-gradient. It is shaped such that the radius of following loops increases perpendicular to the field relevant conductors 21 (here the central conductors 21 with the arrow pointing to the left) with a non-constant distance between adjacent conductors. Due to the V-shape of the gradient system, the center of following loops moves to the center and to the right. Thus, the radius of a set of loops increases in X-direction as well as in Y-direction with the outer conductors 21 essentially remaining at the front side of the gradient system.

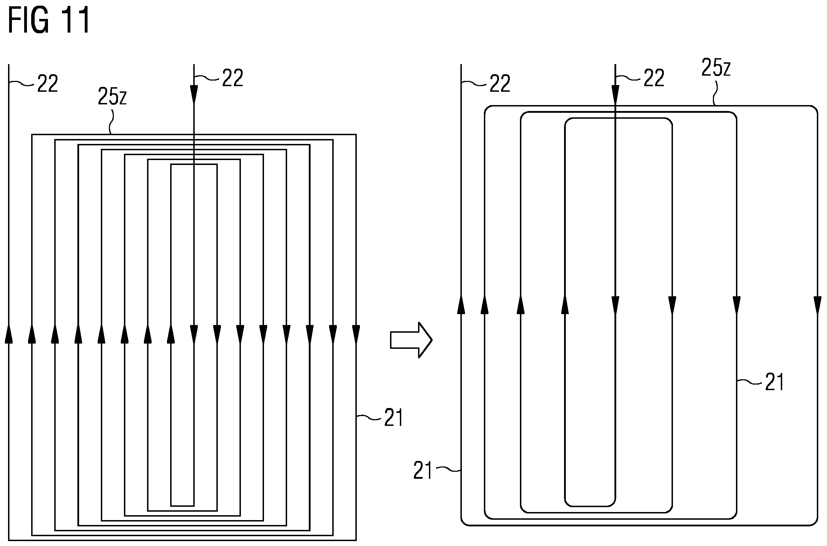

FIG 11 shows an alternative winding for a gradient coil 25z for the Z-gradient. It is shaped such that the radius of following loops increases perpendicular to the field relevant conductors 21 (here the central conductors 21 with the arrow pointing up and down) with a non-constant distance between adjacent conductors. The center of following loops stay essentially in the center of the gradient coil 25z. Thus, the distance of the field-relevant conductors of a set of loops steadily decreases at least in the direction to the front side of the gradient system.

The initial patterns shown in figures 6 to 11 can be further optimized by hardware or software tools to satisfy additional constrains. With the wire patterns as depicted by figures 9 to 11, the generated gradient fields would be slightly non-linear along a radial direction or along the local X-axis. This is not a serious limitation for the use of this solution in prior-art MRI-systems, as the prior art of MRI provides various image reconstruction methods that accommodate for non-linear gradient fields. Additionally, MR imaging using non-linear gradients may provide further advantages as variable spatial resolution, faster data acquisition with non-linear signal encoding and feebler peripheral nerves stimulation.

The alternative gradient coils 25x, 25y, 25z depicted at the right side of figures 9 to 11 are optimized to generate linearly varying gradient fields also along the radial/local x-axis.

Although the present invention has been disclosed in the form of preferred embodiments and variations thereon, it will be understood that numerous additional modifications and variations could be made thereto without departing from the scope of the invention. For the sake of clarity, it is to be understood that the use of "a" or "an" throughout this application does not exclude a plurality, and "comprising" does not exclude other steps or elements. The mention of a "unit" or a "device" does not preclude the use of more than one unit or device.

## Claims

1. A gradient system (20) for a magnetic resonance imaging system comprising a number of pairs of gradient coils (25x, 25y, 25z), wherein the gradient coils (25x, 25y, 25z) of each pair comprise a central plane (23) and are arranged at opposite sides of an examination area (M1, M2, M3, M4, M5, M6) such that the central planes (23) of the two gradient coils (25x, 25y, 25z) of a pair are at an angle greater than 10° to another so that the gradient system (20) is V-shaped.

2. The gradient system according to claim 1, wherein the gradient coils (25x, 25y, 25z) of each pair are arranged in a mirrored arrangement at the opposite sides of the examination area (M1, M2, M3, M4, M5, M6) such that the central planes (23) of the two gradient coils (25x, 25y, 25z) of a pair are symmetrical to an angle bisector.

3. The gradient system according to one of the preceding claims, wherein the gradient coils (25x, 25y, 25z) are bi-planar gradient coils (25x, 25y, 25z),
preferably wherein the central planes (23) of gradient coils (25x, 25y, 25z) on one side of the examination area (M1, M2, M3, M4, M5, M6) are parallel to each other.

4. The gradient system according to one of the preceding claims, wherein a number of gradient coils (25x, 25y, 25z) is formed as local coils, preferably as local gradient coils (25x, 25y, 25z) for head imaging
and/or
wherein a number of gradient coils (25x, 25y, 25z) is formed to cover a side of a whole field-of-view of an examination area (M1, M2, M3, M4, M5, M6), preferably being mechanically and/or permanently attachable to an MRI-scanner (2).

5. The gradient system according to one of the preceding claims, comprising magnetic field shim coils and/or active shielding coils, wherein these coils are preferably arranged parallel to the gradient coils (25x, 25y, 25z).

6. The gradient system according to one of the preceding claims, wherein a gradient coil (25x, 25y, 25z) is formed by a plurality of loops of a conductor (21), preferably wherein
- the loops of a gradient coil (25x, 25y, 25z) for the X-gradient comprising two sets of contrarotating loops adjacent in X-direction, preferably wherein the radius of a set of loops increases, with the outer conductors (21) in X-direction essentially remaining at the sides of the gradient coil (25x, 25y, 25z),
- the loops of a gradient coil (25x, 25y, 25z) for the Y-gradient comprising two sets of contrarotating loops adjacent in Y-direction, preferably wherein the radius of a set of loops increases, with the outer conductors in Y-direction essentially remaining at the sides of the gradient coil (25x, 25y, 25z),
- the loops of a gradient coil (25x, 25y, 25z) for the Z-gradient comprising a set of increasing loops, preferably wherein the center of the loops essentially remain in the center of the gradient coil (25x, 25y, 25z).

7. The gradient system according claim 6, wherein
regarding the gradient coil (25x, 25y, 25z) for the X-gradient and/or the gradient coil (25x, 25y, 25z) for the Z-gradient, the distance of the field-relevant conductors (21) of a set of loops steadily decreases at least in the direction of the aperture of the V-shape of the gradient system (20), and/or
wherein regarding the gradient coil (25x, 25y, 25z) for the Y-gradient, also the radius of a set of loops increases in X-direction to the aperture of the V-shape with the outer conductors essentially remaining at the aperture.

8. A magnetic resonance imaging system (1) comprising at least one examination area (M1, M2, M3, M4, M5, M6), preferably two or more examination areas (M1, M2, M3, M4, M5, M6), and a gradient system (20) according to one of the preceding claims in at least one examination area (M1, M2, M3, M4, M5, M6) .

9. The magnetic resonance imaging system according to claim 8, wherein the gradient system comprises a number of gradient coils (25x, 25y, 25z) covering a side of a whole field-of-view of an examination area (M1, M2, M3, M4, M5, M6), preferably being mechanically and/or permanently attached to the an MRI-scanner (2) of the magnetic resonance imaging system (1).

10. The magnetic resonance imaging system according to claim 8 or 9, wherein central planes (23) of gradient coils (25x, 25y, 25z) of the gradient system (20) on at least one side of the examination area (M1, M2, M3, M4, M5, M6) are parallel to basic field magnets of a MRI-scanner (2) of the magnetic resonance imaging system (1).

11. The magnetic resonance imaging system according to one of claims 8 to 10, wherein the magnetic resonance imaging system (1) is designed such that the gradient system (20) in an examination area (M1, M2, M3, M4, M5, M6), preferably in each examination area (M1, M2, M3, M4, M5, M6) operates asynchronously and/or independent of the gradient system (20) in another examination area (M1, M2, M3, M4, M5, M6) of the magnetic resonance imaging system (1).

12. The magnetic resonance imaging system according to one of claims 8 to 11, comprising a central control unit (13) that is designed to coordinate all gradient activities, preferably the independent operation of different gradient systems (20), especially even the minimization and/or the correction of cross-interference terms between the gradient systems (20).
